# EUROPEAN PATENT SPECIFICATION

(11) **EP 2 454 997 B1**
(45) Date of publication and mention of the grant of the patent: **27.09.2017**
(21) Application number: 11189008.3
(22) Date of filing: 14.11.2011
(51) Int. Cl.: A61B 8/08, G01S 7/52

(54) **Method, device and program for analyzing cartilage using ultrasonic wave**
Verfahren, Vorrichtung und Programm zur Knorpelanalyse mit Ultraschallwellen
Procédé, dispositif et programme d'analyse de cartilage par ondes ultrasonores

(30) Priority: 18.11.2010 JP 2010257403
(43) Date of publication of application: 23.05.2012
(73) Proprietor: Furuno Electric Company Limited, Hyogo (JP)
(72) Inventor: Kiyan, Wataru, Nishinomiya City, Hyogo (JP)
(74) Representative: Williams, Michael Ian

(56) References cited:
- WO-A1-00/36433
- WO-A1-95/10229
- JP-A- 2004 321 582
- US-A- 4 763 661
- US-A- 5 320 102
- US-A- 5 603 326
- US-A1- 2003 032 880
- US-A1- 2003 065 264
- US-A1- 2005 240 089
- US-B1- 6 539 328
- KIVIRANTA P ET AL: "Differences in Acoustic Properties of Intact and Degenerated Human Patellar Cartilage During Compression", ULTRASOUND IN MEDICINE AND BIOLOGY, NEW YORK, NY, US, vol. 35, no. 8, 1 August 2009 (2009-08-01) , pages 1367-1375, XP026321836, ISSN: 0301-5629, DOI: 10.1016/J.ULTRASMEDBIO.2009.03.003 [retrieved on 2009-06-21]
- WILSON A ET AL: "Development of automated ultrasonic measurements of articular cartilage thickness and surface morphology", ENGINEERING IN MEDICINE AND BIOLOGY SOCIETY, 1993. PROCEEDINGS OF THE 15TH ANNUAL INTERNATIONAL CONFERENCE OF THE IEEE OCT 28-31, 1993, PISCATAWAY, NJ, USA,IEEE, 28 October 1993 (1993-10-28), pages 1122-1123, XP010574737, ISBN: 978-0-7803-1377-4

## Description

### Technical Field

The present invention relates to an ultrasonic cartilage analyzing device, an ultrasonic cartilage analyzing method, and a program that provides quantitive information for analyzing a state of a cartilage.

### Background Art

Conventionally, in order to evaluate a state of a cartilage in an articular (joint) cavity intuitively and quantitatively, various systems, which applies an ultrasonic wave to the cartilage and generates analysis data based on a reflection echo of the ultrasonic wave, have been devised.

For example, JP2002-345821A discloses an ultrasonic analysis system in which an endoscope provided with an ultrasonic transceiving body at a tip end thereof is inserted into a joint, and an echo of a pulse signal transmitted from the ultrasonic transceiving body is acquired. Then, a wavelet conversion of the echo signal is carried out, and a cartilage thickness result calculated by a cartilage surface echo level, a cartilage surface echo pulse width, and an assumed acoustic velocity is displayed.

However, when the method disclosed in JP2002-345821A is used as it is for a transcutaneous measurement, since echo amplitude is influenced by soft tissues, it is difficult to evaluate an absolute quantity of the amplitude.

Kiviranta P et al "Differences in acoustic properties of intact and degenerated human patellar cartilage during compression" describes a high resolution material testing device. Cartilage discs can be placed between a metal plate and an ultrasound transducer. Reflected ultrasonic waves are used to analyse the cartilage discs.

WO95/10229A1 describes a device for non-invasive quantitative evaluation of musculoskeletal tissue performed in-vivo. The tissue is placed between two ultrasonic transducers. The device relies on multivariate nonlinear analysis to determine characteristics of the tissue.

US5603326A describes a method for producing an ultrasound image from received ultrasound echo signals particularly for use in the diagnosis of breast cancer. The method relies on detecting a variation in amplitude and/or transit time of signals reflected from healthy and tumorous tissue.

US4763661A describes a device that uses ultrasonic waves to detect diseased tissue by analysing the amplitude distribution of a filtered backscattered ultrasound signal.

JP2004-321582A describes an ultrasonic diagnostic device. The device aims to minimise errors by generating improved statistical data.

Wilson et al: "Development of automated ultrasonic measurements of articular cartilage thickness and surface morphology" describes a method of measuring static and dynamic dimensions of articular cartilage. A pulse-echo ultrasonic system is used to generate short bursts of sound for analysing the cartilage.

WO00/36433A1 describes a process for clinical examination of arteries to assist non-invasive diagnosis of arterial abnormalities.

US6539328B1 describes a device for measurement and treatment of spinal mobility. A head part applies a force impulse to a spinal segment and the subsequent reflected waveform is analysed. The device includes an inclinometer which determines the angle of incidence of the head part in contact with the spinal segment.

### Disclosure of the Invention

Therefore, the present invention is made in view of the above situation, and provides an ultrasonic cartilage analyzing device, an ultrasonic cartilage analyzing method, and a program that can provide analysis data for evaluating accurate cartilaginous properties quantitatively based on spatial feature amounts of a cartilaginous echo.

According to a first aspect of the present invention there is provided an ultrasonic cartilage device as defined in claim 1.

According to a second aspect of the present invention there is provided a method of analyzing a cartilage as defined in claim 8.

According to a third aspect of the invention there is provided a computer readable program as defined in claim 9.

Preferred features of the invention are recited in the dependent claims.

With this configuration, the analysis data relating to the cartilage state of the living body is generated based on the statistical value(s) of the amplitude values of the two or more echo signals reflected on the cartilage surface. By using the statistical value(s) of the amplitude values of the two or more echo signals for generation of the analysis data, the analysis data for quantitatively evaluating an accurate state of the cartilage through a skin of the living body can be provided.

The calculator may calculate a variation coefficient as the statistical value based on the amplitude values acquired by the acquisition unit.

This configuration shows the use of the variation coefficient as a calculation example of the statistical value of the amplitude values.

The acquisition unit may acquire the amplitude values of the two or more echo signals from the cartilage surface or a surface substantially parallel with the cartilage surface. The calculator may calculate the statistical value of the amplitude values of the cartilage surface or the surface substantially parallel with the cartilage surface.

This configuration shows a calculation example of the acquisition of the amplitude values of the echo signals, and the statistical value of the amplitude values.

The acquisition unit may acquire the amplitude value of the echo signal at any time. The calculator may calculate the statistical value of the amplitude values along a normal direction of the cartilage surface.

This configuration shows an example of the statistical value of the amplitude values to calculate.

The ultrasonic cartilage analyzing device may further include a transmitter for transmitting a pulse burst signal to different positions of the living body.

With this configuration, the pulse burst signal is used as an example of the ultrasonic signal transmitted to the living body.

The ultrasonic cartilage analyzing device may further include a display unit for displaying the analysis data generated by the generator.

This configuration shows a configuration which can display the analysis data.

The selecting unit may select the ultrasonic signal based on time periods, and each time period may be between a transmission of the ultrasonic signal and reception of the echo signal.

According to the present invention, quantitive analysis data which cannot be easily influenced by soft tissue can be provided using the statistical value(s) of the amplitude values of the two or more echo signals for the generation of the analysis data.

### Brief Description of the Drawings

The present disclosure is illustrated by way of example and not by way of limitation in the figures of the accompanying drawings, in which the like reference numerals indicate like elements and in which:
Fig. 1 is a block diagram showing a configuration of an ultrasonic cartilage analyzing device according to one embodiment of the present invention;
Fig. 2 is a view schematically showing an area, where an oscillator transmits a pulse burst signal, such as a person's knee viewed from front;
Fig. 3 is a view showing analysis data in the case of a normal cartilage;
Fig. 4 is a view showing analysis data in the case of an initial degenerated cartilage;
Fig. 5 shows graphs of analysis data in which a change of a variation coefficient CV in a depth direction is shown;
Fig. 6 shows graphs of analysis data in which the change of the variation coefficient CV in the depth direction is shown;
Fig. 7 shows graphs of analysis data in which the change of the variation coefficient CV in the depth direction is shown; and
Fig. 8 is a flowchart showing a procedure of processing performed with the ultrasonic cartilage analyzing device.

### Detailed Description

Hereinafter, a suitable embodiment of an ultrasonic cartilage analyzing device, an ultrasonic cartilage analyzing method, and a program according to the present invention is described with reference to the accompanying drawings. In this embodiment, although a cartilage of person's knee is described as an example of a sample for the ultrasonic cartilage analyzing device, the following configuration of this embodiment is also applicable to other devices for analyzing an internal structure of the sample from outside the sample, similarly using an ultrasonic signal.

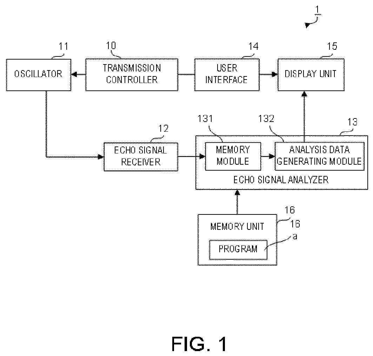

Fig. 1 is a block diagram showing a configuration of the ultrasonic cartilage analyzing device according to this embodiment. The ultrasonic cartilage analyzing device 1 includes a transmission controller 10, an oscillator 11, an echo signal receiver 12, an echo signal analyzer 13, a user interface 14, a display unit 15, and a memory unit 16. The memory unit 16 is, for example, a ROM (Read Only Memory) which stores a necessary program, data, etc.

The user interface 14 sets transmitting parameters, such as a transmitting frequency, a pulse width, or an input voltage, according to an user's input about transmission, and outputs the parameters to the transmission controller 10. The user interface 14 outputs an instruction for setting or switching a display mode to the display unit 15 according to a user's input about displaying. Note that the user interface 14 may be incorporated in the display unit 15.

The transmission controller 10 generates a drive signal for transmitting an ultrasonic signal to the oscillator 11 (described in detail below) according to the frequency setting and a transmission timing interval. The frequency and the transmission timing interval may be set by a user via the user interface 14, or may be set in advance. The transmission controller 10 outputs the generated drive signal to the oscillator 11.

The oscillator 11 (transmitter) is housed in a case suitable for being used while contacting an outer skin of the knee (for example, similar to a case of a handy scanner). The oscillator 11 transmits the ultrasonic signal with a predetermined directivity by the drive signal from the transmission controller 10. Then, the oscillator 11 receives an echo signal which is a reflection of the transmitted ultrasonic signal from the surface of the knee cartilage, converts the echo signal into an electric signal, and outputs it to the echo signal receiver 12. Note that, the term "cartilage surface" as used herein refers to a cross section of the cartilage at a distance (depth) set from the knee outer skin. The depth may be set by the user via the user interface 14, or may be set in advance.

Below, the ultrasonic signal transmitted from the oscillator 11 refers to a pulse burst signal which is relatively long in a pulse length. The pulse burst signal means a pulse signal of a waveform which is formed by continuously transmitting a carrier wave at the frequency setting for a period of a predetermined number of waves (for example, five waves). Note that, the transmitting ultrasonic signal may also be a short pulse signal of a relatively short pulse length according to specifications of the device or a situation the device is used.

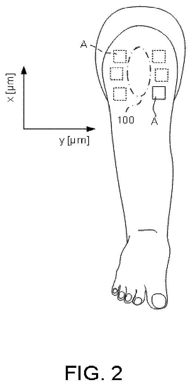

Fig. 2 is a schematic diagram showing a state in which an area to which the oscillator 11 transmits the pulse burst signal is set to a person's knee. Fig. 2 is a front view of the knee which is bent. The area A to which the oscillator 11 transmits the pulse burst signal (hereinafter, referred to as an "attention area") is a two-dimensional area of about 1 to 2 cm (i.e., about 1 cm x 1 cm square to about 2 cm x 2 cm square), and is set at a location where a patella 100 shown by a dashed dotted line in Fig. 2 is avoided.

The attention area A may be a location where the patella 100 is excluded, and, therefore, it may be a portion shown by a solid line in Fig. 2, or may be a portion shown by a dashed line. The oscillator 11 of this embodiment transmits a pulse burst signal to two or more locations within the attention area A substantially perpendicularly to the attention area A, and receives the echo signals. That is, since the attention area A is substantially parallel with the cartilage surface, the oscillator 11 transmits and receives two or more signals two-dimensionally on a surface parallel with the cartilage surface. Below, as shown in Fig. 2, a length direction of the leg is set as x-axis direction and a width direction as y-axis direction. Note that the oscillator 11 is configured to scan throughout the attention area A. The scanning may be mechanical scanning or electronic scanning.

The transmission controller 10 and the oscillator 11 can transmit the pulse burst signal at two or more different transmitting frequencies. For example, the transmission controller 10 and the oscillator 11 can transmit the pulse burst signal at a specific frequency within a predetermined frequency band, such as about 10MH to 24MHz. Here, a reception period is set longer than a period from a timing at which the pulse burst signal is transmitted to the person's knee to a timing at which the oscillator 11 receives the reflection signal of the pulse burst signal which reached to the predetermined depth. The frequency of the pulse burst signal may be set in advance as a frequency suitable for acquiring echo data (described later) suitable for an evaluation of person's cartilage or analysis data for the cartilage, using the results obtained from experiments. Alternatively, the frequency may be set as a frequency which is adjusted by the user via the user interface 14 after acquiring analysis data of an echo pattern due to the pulse burst signals at two or more frequencies.

The echo signal receiver 12 creates data from the echo signal at a predetermined sampling timing to form the echo data where individual echo data sampled along the depth direction (normal direction) from the knee surface is allocated sequentially. The echo signal receiver 12 forms the echo data at two or more locations within the attention area A where the pulse burst signal is transmitted. That is, the echo data corresponding to one pulse burst signal (hereinafter, referred to as "echo data of 1PING") is comprised of a group of the individual echo data allocated sequentially at a predetermined interval along the depth direction, and the two or more echo data are formed corresponding to the attention area A along the cartilage surface. The echo signal receiver 12 outputs the formed echo data to the echo signal analyzer 13.

The echo signal analyzer 13 is comprised of a microcomputer, for example, which executes a program 16a stored in the memory unit 16 to generate the analysis data for cartilage using the echo data inputted from the echo signal receiver 12. The echo signal analyzer 13 includes a memory module 131 and an analysis data generating module 132.

The memory module 131 has a capacity for storing the two or more echo data for the predetermined number of PINGs to sequentially store the echo data for every PING inputted from the echo signal receiver 12. Under the present circumstances, when new echo data is inputted in a case where the echo data is stored to the limit of the storage capacity, processing which writes new echo data over the echo data of the oldest PING is performed. Thereby, the echo data for the newest predetermined PINGs are stored in the memory module 131.

The analysis data generating module 132 acquires the echo data from the memory module 131 at any time, and generates the analysis data for the cartilage at the predetermined depth. The generated analysis data is displayed on the display unit 15 where properties of the cartilage are evaluated.

Below, the analysis data generated by the analysis data generating module 132 is described.

The analysis data generating module 132 acquires an amplitude value of the echo signal at the predetermined depth from each echo data within the attention area A, and generates a distribution map of the amplitude values along the cartilage surface. Next, the analysis data generating module 132 calculates an average value and a standard deviation of the amplitude values of the attention area A from the acquired amplitude values, respectively, and, further calculates a variation coefficient CV (statistical value). The variation coefficient CV is calculated based on the standard deviation/average value. The analysis data generating module 132 generates the distribution map of the generated amplitude values, and the analysis data containing the calculated variation coefficient CV.

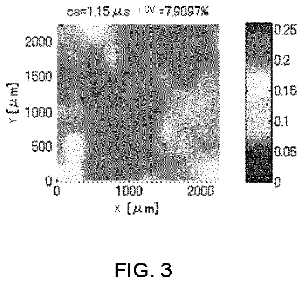

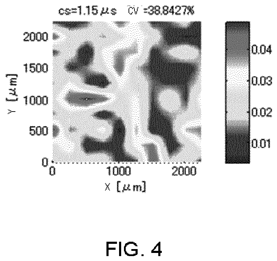

Fig. 3 is a view showing the analysis data in the case of a normal cartilage. On the other hand, Fig. 4 is a view showing the analysis data in the case of being a cartilage where early denaturation started (hereinafter, referred to as an "initial degenerated cartilage"). The initial degenerated cartilage refers to an articular cartilage where the surface collagen density is reduced. Figs. 3 and 4 show the distribution map of the amplitude values of the echo signals reflected on the cartilage surface within the attention area A shown in Fig. 2, and the calculated variation coefficient CV of the attention area A, respectively. The right-hand sides of Figs. 3 and 4 show that it becomes white as the amplitude value becomes larger and it becomes black as the amplitude value becomes smaller.

Since in the case of the normal cartilage the collagen density of the surface which constitutes the cartilage is large and uniform, the amplitude value of the normal cartilage surface is substantially uniform (see Fig. 3). On the other hand, in the case of the initial degenerated cartilage, since the non-uniformity of the surface collagen density increases in the process of collagen decomposition and the variation in acoustic reflectance becomes larger, the variation in the amplitude value also becomes larger (see Fig. 4). For this reason, the analysis data generating module 132 calculates the variation coefficient CV which the variation in this amplitude value reflects. The variation in the amplitude value becomes smaller as the variation coefficient CV becomes smaller. That is, when the cartilage is normal, the variation in the amplitude value becomes larger as the variation coefficient CV becomes larger. That is, it is in a state where the denaturation of the cartilage has been progressed. Therefore, as shown in Figs. 3 and 4, the variation coefficient CV of the normal cartilage becomes smaller than the variation coefficient CV of the degenerated cartilage. Thus, the evaluation of the cartilage properties is possible by using the variation coefficient CV reflecting the variation in the amplitude value of two or more locations on the cartilage surface at the predetermined depth, without using an absolute level of the amplitude value.

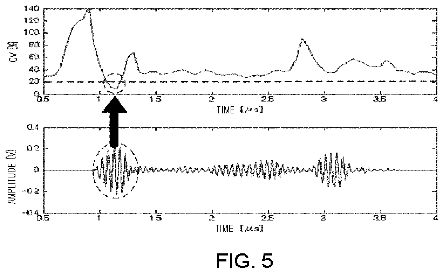

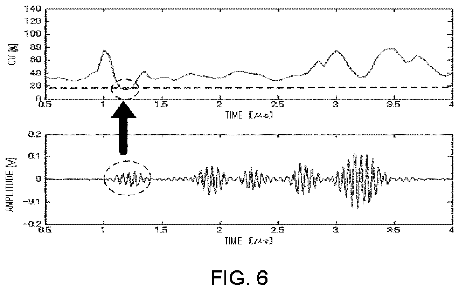

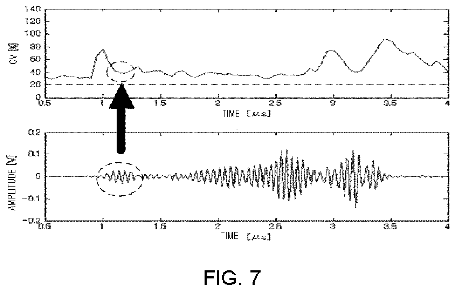

Note that the analysis data generating module 132 may generate analysis data indicative of a change of the variation coefficient CV in the depth direction. Figs. 5, 6, and 7 are graphs of the analysis data indicative of the change of the variation coefficient CV in the depth direction. In Figs. 5, 6, and 7, the change of the variation coefficient CV with respect to the depth, and the amplitude value of the echo signal are shown in the upper graph and the lower graph, respectively, where a time period until the echo signal is received (i.e., depth) is taken as the horizontal axis, respectively. Fig. 5 shows the case of the normal cartilage, and Figs. 6 and 7 show the cases of the initial degenerated cartilage in this order.

Compared with Fig. 5, the variation coefficient CV is high at the cartilage surface as denaturation progresses in Figs. 6 and 7 (about 1.1 microseconds). In this temporal change of the variation coefficient CV, by focusing on the change of the variation coefficient CV from the cartilage surface to the inside thereof, it becomes possible to evaluate the properties of the cartilage surface. In addition, a threshold of the variation coefficient CV shown by the dotted line in each drawing is set (for example, about 20%), and it also becomes possible to evaluate the properties of the cartilage surface based on the existence of an area where the variation coefficient CV is less than the threshold (an dotted ellipse portion in the drawing). If there is an area where the coefficient is less than the threshold (see Fig. 5), the cartilage of the attention area A is a normal cartilage, and if there is no area where the coefficient is less than the threshold (see Fig. 7), it is estimated that the cartilage of the attention area A is an initial degenerated cartilage.

Alternatively, the analysis data generating module 132 may generate analysis data where the variation in the amplitude value within the attention area A is converted into a histogram. Alternatively, the analysis data generating module 132 may generate the analysis data where the distribution map of the amplitude values at two or more positions in the depth direction is applied a two-dimensional Fourier transformation. In this case, it becomes possible to evaluate the cartilage properties by evaluating the concavo-convex cycle or discontinuity in the pattern after the conversion.

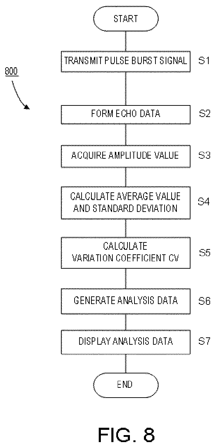

Next, an operation of the ultrasonic cartilage analyzing device 1 is described. Fig. 8 is a flowchart showing a procedure 800 of processing executed with the ultrasonic cartilage analyzing device 1.

The transmission controller 10 of the ultrasonic cartilage analyzing device 1 generates the drive signal, and causes the oscillator 11 to transmit the pulse burst signal to the attention area A (S1). Next, the echo signal receiver 12 forms the echo data according to the transmitted pulse burst signal (S2), and the echo signal analyzer 13 acquires the amplitude value of the echo signal within the attention area A based on the echo data (S3).

The echo signal analyzer 13 calculates the average value and the standard deviation of the amplitude values within the attention area A based on the acquired amplitude value (S4), and then calculates the variation coefficient CV based on the average value and the standard deviation (S5). Then, the echo signal analyzer 13 generates the analysis data shown in Figs. 3 and 4 based on the acquired amplitude value and the calculated variation coefficient CV (S6). The echo signal analyzer 13 outputs the generated analysis data to the display unit 15, and the display unit 15 then displays the result (S7). Based on the result displayed on the display unit 15, the evaluation of the properties of the cartilage within the attention area A is performed.

As described above, in this embodiment, the ultrasonic cartilage analyzing device 1 generates the analysis data for cartilage indicative of the variation coefficient CV (fluctuation) of the amplitude value of the echo signal within the attention area A. Thereby, information for evaluating the existence of the degenerated cartilage can be provided to a user visually, quantitatively and clearly, without detecting the absolute level of the amplitude value.

Note that, in the above embodiment, the echo signal receiver 12 forms the echo data based on all the echo signals received by the oscillator 11, it may form the echo data only based on a part of the echo signal received by the oscillator 11.

The cartilage surface is irregular. Therefore, even if the oscillator 11 transmits the signal perpendicular to the attention area A which is substantially parallel with the cartilage surface, it is difficult for the transmitted signal to actually enter into the cartilage surface perpendicularly.

For this reason, the oscillator 11 determines whether the incident angle of the signal on the cartilage surface is below a predetermined permissible incident angle based on two or more echo signals which are received by scanning the attention area A. The incident angle on the cartilage surface can be calculated based on a time period until the signal transmitted from the oscillator 11 arrives at the cartilage surface. For example, by comparing between the time periods until the oscillator 11 receives the echo signals at three locations, which are close to each other, within the attention area A (arrival times), the echo signal receiver 12 can determine whether the incident angle on the cartilage surface is substantially perpendicular.

The permissible incident angle can be set based on an angle range which falls under an allowable sensitivity, in terms of reception directional characteristics of the oscillator 11. For example, when the allowable sensitivity of the oscillator 11 is less than -6 [dB], the permissible incident angle will be set as 5 degrees if the angle characteristic is ±5 degrees at -6 [dB]. The permissible incident angle is not limited to this, but may be changed suitably.

By the above, since the echo data can be formed only from a reliable echo signal, the properties of a cartilage can be evaluated with more sufficient accuracy.

In the foregoing specification, specific embodiments of the present invention have been described. Accordingly, the specification and figures are to be regarded in an illustrative rather than a restrictive sense, and all such modifications are intended to be included within the scope of present invention. The benefits, advantages, solutions to problems, and any element(s) that may cause any benefit, advantage, or solution to occur or become more pronounced are not to be construed as a critical, required, or essential features or elements of any or all the claims. The invention is defined solely by the appended claims including any amendments made during the pendency of this application and all equivalents of those claims as issued.

Moreover in this document, relational terms such as first and second, top and bottom, and the like may be used solely to distinguish one entity or action from another entity or action without necessarily requiring or implying any actual such relationship or order between such entities or actions. The terms "comprises," "comprising," "has," "having," "includes," "including," "contains," "containing" or any other variation thereof, are intended to cover a non-exclusive inclusion, such that a process, method, article, or apparatus that comprises, has, includes, contains a list of elements does not include only those elements but may include other elements not expressly listed or inherent to such process, method, article, or apparatus. An element proceeded by "comprises ...a," "has ...a," "includes ...a," "contains ...a" does not, without more constraints, preclude the existence of additional identical elements in the process, method, article, or apparatus that comprises, has, includes, contains the element. The terms "a" and "an" are defined as one or more unless explicitly stated otherwise herein. The terms "substantially," "essentially," "approximately," "about" or any other version thereof, are defined as being close to as understood by one of ordinary skill in the art, and in one non-limiting embodiment the term is defined to be within 10%, in another embodiment within 5%, in another embodiment within 1% and in another embodiment within 0.5%. The term "coupled" as used herein is defined as connected, although not necessarily directly and not necessarily mechanically. A device or structure that is "configured" in a certain way is configured in at least that way, but may also be configured in ways that are not listed.

## Claims

1. An ultrasonic cartilage analyzing device (1) for generating analysis data relating to a state of a cartilage of a living body based on two or more echo signals that are reflections of two or more ultrasonic signals transmitted from outside the living body to different positions inside the living body, the device comprising:
a selecting unit (12) configured to select ultrasonic signals of which an incident angle on the cartilage surface is substantially perpendicular, from the two or more transmitted ultrasonic signals;
an acquisition unit (13) configured to acquire amplitude values of two or more echo signals reflected on the surface of the cartilage corresponding to the ultrasonic signals selected by the selecting unit (12);
a calculator (13) configured to calculate a statistical value of the amplitude values acquired by the acquisition unit; and
a generator (13) configured to generate the analysis data relating to the state of the cartilage based on the statistical value calculated by the calculator.

2. The ultrasonic cartilage analyzing device (1) of Claim 1, wherein the calculator (13) is configured to calculate a variation coefficient as the statistical value based on the amplitude values acquired by the acquisition unit (13).

3. The ultrasonic cartilage analyzing device (1) of Claim 1 or 2, wherein the acquisition unit (13) is configured to acquire the amplitude values of the two or more echo signals from the cartilage surface or a surface substantially parallel with the cartilage surface, and
wherein the calculator (13) is configured to calculate the statistical value of the amplitude values of the cartilage surface or the surface substantially parallel with the cartilage surface.

4. The ultrasonic cartilage analyzing device (1) of Claim 1 or 2, wherein the acquisition unit (13) is configured to acquire the amplitude value of the echo signal at any time, and
wherein the calculator (13) is configured to calculate the statistical value of the amplitude values along a normal direction of the cartilage surface.

5. The ultrasonic cartilage analyzing device (1) of any one of Claims 1 to 4, further comprising a transmitter (11) for transmitting a pulse burst signal to different positions of the living body.

6. The ultrasonic cartilage analyzing device (1) of any one of Claims 1 to 5, further comprising a display unit (15) for displaying the analysis data generated by the generator (13).

7. The ultrasonic cartilage analyzing device of any of the preceding claims, wherein the selecting unit (12) selects the ultrasonic signal based on time periods, each time period being between a transmission of the ultrasonic signal and a reception of the echo signal.

8. A method (800) of analyzing a cartilage of a living body using ultrasonic signals, the method generating analysis data relating to a state of the cartilage based on two or more echo signals that are reflections of two or more ultrasonic signals transmitted from outside the living body to different positions inside the living body, the method comprising:
selecting (s2) ultrasonic signals of which an incident angle on the cartilage surface is substantially perpendicular, from the two or more transmitted ultrasonic signals;
acquiring (s3) amplitude values of two or more echo signals reflected on the surface of the cartilage corresponding to the selected ultrasonic signals;
calculating (s4) a statistical value of the acquired amplitude values; and
generating (s6) the analysis data relating to the state of the cartilage based on the calculated statistical value.

9. A computer readable program (800) for causing a computer (1) to analyze a cartilage of a living body using ultrasonic signals, the program causing a computer to generate analysis data relating to a state of the cartilage based on two or more echo signals that are reflections of two or more ultrasonic signals transmitted from outside the living body to different positions inside the living body, comprising:
causing a computer (1) to select (s2) ultrasonic signals of which an incident angle on the cartilage surface is substantially perpendicular, from the two or more transmitted ultrasonic signals;
causing a computer (1) to acquire (s3) amplitude values of two or more echo signals reflected on the surface of the cartilage corresponding to the selected ultrasonic signals;
causing a computer (1) to calculate (s4) a statistical value of the acquired amplitude values; and
causing a computer (1) to generate (s6) the analysis data relating to the state of the cartilage based on the calculated statistical value.

## Patentansprüche

1. Ultraschallknorpeluntersuchungsgerät (1) zum Generieren von Untersuchungsdaten, die sich auf einen Zustand eines Knorpels eines lebenden Körpers beziehen, basierend auf zwei oder mehreren Echosignalen, die Reflektionen zweier oder mehrerer Ultraschallsignale sind, die von außerhalb des lebenden Körpers zu verschiedenen Positionen innerhalb des lebenden Körpers übertragen wurden, das Gerät umfassend:
eine Auswahleinheit (12), die eingerichtet ist, um Ultraschallsignale, bei welchen ein Einfallwinkel auf der Knorpeloberfläche im Wesentlichen senkrecht ist, aus den zwei oder mehr übertragenen Ultraschallsignalen auszuwählen;
eine Erfassungseinheit (13), die eingerichtet ist, um Amplitudenwerte zweier oder mehrerer Echosignale, die auf der Oberfläche des Knorpels reflektiert wurden, entsprechend den von der Auswahleinheit (12) ausgewählten Ultraschallsignalen zu erfassen;
ein Rechner (13), der eingerichtet ist, um einen statistischen Wert der Amplitudenwerte zu berechnen, die von der Erfassungseinheit erfasst wurden; und
einen Generator (13), der eingerichtet ist, um die Untersuchungsdaten, die sich auf den Zustand des Knorpels beziehen, basierend auf dem statistischen Wert, der von dem Rechner berechnet wurde, zu generieren.

2. Ultraschallknorpeluntersuchungsgerät (1) nach Anspruch 1, wobei der Rechner (13) eingerichtet ist, um einen Variationskoeffizienten als den statistischen Wert basierend auf den Amplitudenwerten, die von der Erfassungseinheit erfasst wurden, zu berechnen.

3. Ultraschallknorpeluntersuchungsgerät (1) nach Anspruch 1 oder 2, wobei die Erfassungseinheit (13) eingerichtet ist, um die Amplitudenwerte der zwei oder mehreren Echosignale von der Knorpeloberfläche oder einer im Wesentlichen zur Knorpeloberfläche parallelen Oberfläche zu erfassen, und
wobei der Rechner (13) eingerichtet ist, um den statistischen Wert der Amplitudenwerte der Krtorpeloberfläche oder der im Wesentlichen zu Knorpeloberfläche parallelen Oberfläche zu berechnen.

4. Ultraschallknorpeluntersuchungsgerät (1) nach Anspruch 1 oder 2, wobei die Erfassungseinheit (13) eingerichtet ist, um die Amplitudenwerte des Echosignals zu jeder Zeit zu erfassen, und
wobei der Rechner (13) eingerichtet ist, um den statistischen Wert der Amplitudenwerte entlang einer Normalenrichtung der Knorpeloberfläche zu berechnen.

5. Ultraschallknorpeluntersuchungsgerät (1) nach einem der Ansprüche 1 bis 4, ferner umfassend einen Sender (11) zum Übertragen eines Burstsignals an verschiedene Positionen des lebenden Körpers.

6. Ultraschallknorpeluntersuchungsgerät (1) nach einem der Ansprüche 1 bis 5, ferner umfassend eine Anzeigeeinheit (15) zum Anzeigen der Untersuchungsdaten, die von dem Generator (13) generiert wurden.

7. Ultraschallknorpeluntersuchungsgerät (1) nach einem der vorherigen Ansprüche, wobei die Auswahleinheit (12) die Ultraschallsignale basierend auf Zeitspannen auswählt, wobei jede Zeitspanne zwischen einer Übertragung des Ultraschallsignals und einem Empfangen des Echosignals ist.

8. Verfahren (800) zum Untersuchen eines Knorpels eines lebenden Körpers unter Verwendung von Ultraschallsignalen, wobei das Verfahren Untersuchungsdaten, die sich auf einen Zustand des Knorpels beziehen, basierend auf zwei oder mehreren Echosignalen, die Reflektionen zweier oder mehrerer Ultraschallsignale sind, die von außerhalb des lebenden Körpers zu verschiedenen Positionen innerhalb des lebenden Körpers übertragen wurden, generiert, das Verfahren umfassend:
Auswählen (s2) von Ultraschallsignalen, bei welchen ein Einfallwinkel auf der Knorpeloberfläche im Wesentlichen senkrecht ist, aus den zwei oder mehreren übertragenen Ultraschallsignalen;
Erfassen (s3) von Amplitudenwerten zweier oder mehrerer Echosignale, die auf der Oberfläche des Knorpels reflektiert wurden, entsprechend den ausgewählten Ultraschallsignalen;
Berechnen (s4) eines statistischen Werts der erfassten Amplitudenwerte; und
Generieren (s6) der Untersuchungsdaten, die sich auf den Zustand des Knorpels beziehen, basierend auf dem berechneten statistischen Wert.

9. Computerlesbares Programm (800) zum Veranlassen eines Computers (1) einen Knorpel eines lebenden Körpers unter Verwendung von Ultraschallsignalen zu untersuchen, wobei das Programm einen Computer veranlasst, Untersuchungsdaten, die sich auf einen Zustand des Knorpels beziehen, basierend auf zwei oder mehreren Echosignalen, die Reflektionen zweier oder mehrerer Ultraschallsignale sind, die von außerhalb des lebenden Körpers zu verschiedenen Positionen innerhalb des lebenden Körpers übertragen wurden, zu generieren, umfassend:
einen Computer (1) veranlassen, Ultraschallsignale, bei welchen ein Einfallwinkel auf der Knorpeloberfläche im Wesentlichen senkrecht ist, aus den zwei oder mehreren übertragenen Ultraschallsignalen auszuwählen (s2);
einen Computer (1) veranlassen, Amplitudenwerte zweier oder mehrerer Echosignale, die auf der Oberfläche des Knorpels reflektiert wurden, entsprechend den ausgewählten Ultraschallsignalen zu erfassen (s3);
einen Computer (1) veranlassen, einen statistischen Wert der erfassten Amplitudenwerte zu berechnen (s4); und
einen Computer (1) veranlassen, die Untersuchungsdaten, die sich auf den Zustand des Knorpels beziehen, basierend auf dem berechneten statistischen Wert zu generieren (s6).

## Revendications

1. Dispositif d'analyse de cartilage à ultrasons (1) pour générer des données d'analyse relatives à l'état d'un cartilage d'un corps vivant sur la base de deux ou plus de deux signaux d'écho qui sont des réflexions de deux ou plus de deux signaux ultrasonores transmis depuis l'extérieur du corps vivant à différentes positions à l'intérieur du corps vivant, le dispositif comprenant :
une unité de sélection (12) configurée pour sélectionner des signaux ultrasonores dont un angle d'incidence sur la surface de cartilage est sensiblement perpendiculaire, par rapport aux deux ou plus de deux signaux ultrasonores transmis ;
une unité d'acquisition (13) configurée pour acquérir des valeurs d'amplitude de deux ou plus de deux signaux d'écho réfléchis sur la surface du cartilage correspondant aux signaux ultrasonores sélectionnés par l'unité de sélection (12) ;
un calculateur (13) configuré pour calculer une valeur statistique des valeurs d'amplitude acquises par l'unité d'acquisition ; et
un générateur (13) configuré pour générer les données d'analyse relatives à l'état du cartilage sur la base de la valeur statistique calculée par le calculateur.

2. Dispositif d'analyse de cartilage à ultrasons (1) selon la revendication 1, dans lequel le calculateur (13) est configuré pour calculer un coefficient de variation en tant que valeur statistique sur la base des valeurs d'amplitude acquises par l'unité d'acquisition (13).

3. Dispositif d'analyse de cartilage à ultrasons (1) selon la revendication 1 ou 2, dans lequel l'unité d'acquisition (13) est configurée pour acquérir les valeurs d'amplitude des deux ou plus de deux signaux d'écho provenant de la surface de cartilage ou d'une surface sensiblement parallèle à la surface de cartilage, et
dans lequel le calculateur (13) est configuré pour calculer la valeur statistique des valeurs d'amplitude de la surface de cartilage ou de la surface sensiblement parallèle à la surface de cartilage.

4. Dispositif d'analyse de cartilage à ultrasons (1) selon la revendication 1 ou 2, dans lequel l'unité d'acquisition (13) est configurée pour acquérir la valeur d'amplitude du signal d'écho à tout moment, et
dans lequel le calculateur (13) est configuré pour calculer la valeur statistique des valeurs d'amplitude le long d'une direction normale de la surface de cartilage.

5. Dispositif d'analyse de cartilage à ultrasons (1) selon l'une quelconque des revendications 1 à 4, comprenant en outre un émetteur (11) pour transmettre un signal en rafale pulsé à différentes positions du corps vivant.

6. Dispositif d'analyse de cartilage à ultrasons (1) selon l'une quelconque des revendications 1 à 5, comprenant en outre une unité d'affichage (15) pour afficher les données d'analyse générées par le générateur (13).

7. Dispositif d'analyse de cartilage à ultrasons selon l'une quelconque des revendications précédentes, dans lequel l'unité de sélection (12) sélectionne le signal ultrasonore sur la base de périodes de temps, chaque période de temps étant comprise entre une transmission du signal ultrasonore et une réception du signal d'écho.

8. Procédé (800) d'analyse d'un cartilage d'un corps vivant utilisant des signaux ultrasonores, le procédé générant des données d'analyse relatives à un état du cartilage sur la base de deux ou plus de deux signaux d'écho qui sont des réflexions de deux ou plus de deux signaux ultrasonores transmis depuis l'extérieur du corps vivant à différentes positions à l'intérieur du corps vivant, le procédé comprenant les étapes consistant à :
sélectionner (s2) des signaux ultrasonores dont un angle d'incidence sur la surface de cartilage est sensiblement perpendiculaire, par rapport aux deux ou plus de deux signaux ultrasonores transmis ;
acquérir (s3) des valeurs d'amplitude de deux ou plus de deux signaux d'écho réfléchis sur la surface du cartilage correspondant aux signaux ultrasonores sélectionnés ;
calculer (s4) une valeur statistique des valeurs d'amplitude acquises ; et
générer (s6) les données d'analyse relatives à l'état du cartilage sur la base de la valeur statistique calculée.

9. Programme lisible par ordinateur (800) pour amener un ordinateur (1) à analyser un cartilage d'un corps vivant en utilisant des signaux ultrasonores, le programme amenant un ordinateur à générer des données d'analyse relatives à un état du cartilage sur la base de deux ou plus de deux signaux d'écho qui sont des réflexions de deux ou plus de deux signaux ultrasonores transmis depuis l'extérieur du corps vivant à différentes positions à l'intérieur du corps vivant, comprenant les étapes consistant à :
amener un ordinateur (1) à sélectionner (s2) des signaux ultrasonores dont un angle d'incidence sur la surface de cartilage est sensiblement perpendiculaire, par rapport aux deux ou plus de deux signaux ultrasonores transmis ;
amener un ordinateur (1) à acquérir (s3) des valeurs d'amplitude de deux ou plus de deux signaux d'écho réfléchis sur la surface du cartilage correspondant aux signaux ultrasonores sélectionnés ;
amener un ordinateur (1) à calculer (s4) une valeur statistique des valeurs d'amplitude acquises ; et
amener un ordinateur (1) à générer (s6) les données d'analyse relatives à l'état du cartilage sur la base de la valeur statistique calculée.
